# EUROPEAN PATENT APPLICATION

(11) **EP 2 607 494 A1**
(43) Date of publication of application: **26.06.2013**
(21) Application number: 11195583.7
(22) Date of filing: 23.12.2011
(51) Int. Cl.: C12Q 1/68, G01N 33/50

(54) **Biomarkers for lung cancer risk assessment**

(71) Applicant: Philip Morris Products S.A., 2000 Neuchâtel (CH)
(72) Inventor: The designation of the inventor has not yet been filed
(74) Representative: Vossius & Partner

(57) **Abstract**

The present invention relates to biomarkers that are indicative for the presence of or disposition for lung cancer and can be used in a diagnostic method for determining the cancer status of a patient. The invention further provides kits for carrying out said methods.

## Description

The present invention relates to biomarkers that are indicative for the presence of or disposition for lung cancer and can be used in a diagnostic method for determining the cancer status of a patient. The invention further provides kits for carrying out said methods.

Lung cancer is one of the most common cancers in the world and is a leading cause of cancer death in men and women in the developed world. Diagnosis of lung cancer is still mainly based on chest radiograph and computed tomography (CT scan). Bronchoscopy or CT-guided biopsy may be used to obtain further information and to identify the tumor type.

In recent years an increasing amount of reports has appeared on biomarkers that may be used for risk assessment, screening, diagnosis, prognosis, and for selection and monitoring of therapies of lung cancer.

For example, Semmens et al, 2005 (WO 2005/098445) disclose protein biomarkers that are differentially present in the samples of patients with lung cancer and in the samples of control subjects and can thus be used in diagnosing lung cancer. The measurement of these markers, alone or in combination, in patient samples, is reported to provide information that can be correlated with a probable diagnosis of lung cancer or a negative diagnosis (e. g., normal or disease-free). All the markers are characterized by molecular weight.

Gold et al, 2011 (WO 20011/031344) disclose a list of 61 biomarkers that can be used alone or in various combinations to diagnose lung cancer.

YIP et al, 2004 (WO 2004/061410) disclose a method for qualifying lung carcinoma status in a subject, which comprises analyzing a biological sample from said subject for a diagnostic level of a biomarker protein, which is differentially present in samples of a subject with lung cancer and a normal subject that is free of lung cancer. YIP et al, 2004 provide a list of about 50 preferred biomarkers that may be used in such a method.

Sungwhan et al, 2007 (US 2007/0264659) disclose an epigenetic marker for lung cancer.

Despite the increasing number of reports on biomarkers for lung cancer detection, there is still a need for identification of alternative biomarker combinations that are predictive for smoke-induced lung cancer development in smokers versus non-smokers and that can be used to distinguish between pre-stages or early stages of lung cancer and advanced stages of lung cancer ("early predictive markers").

This need could be met within the scope of the present invention.

The present invention provides biomarkers or a panel of biomarkers that are predictive for smoke-induced lung cancer development and that can be used to develop tools in order to decrease adverse health effects caused by exposure to tobacco smoke and other tobacco-related products. In particular, the present invention provides biomarkers, which were identified based on gene expression patterns that are associated with smoke-induced lung cancer development and can be used to distinguish between pre-stages or early stages of lung cancer and advanced stages of lung cancer ("early predictive markers").

In one embodiment, the present invention thus provides a method for determining in an individual lung cancer or a predisposition for lung cancer, said method comprising determining in a test sample taken from said individual differential expression of a panel of at least 2 and up to 50 different biomarkers selected from the biomarkers depicted in Table 1, Table 2, or both Table 1 and Table 2, particularly when compared to a control sample.

In one embodiment of the invention, the differential expression of the at least two, and up to 50, biomarkers in the test sample is determined to have changed relative to that in the control sample by more than the 3-fold, 3.5 fold, 4.0 fold, 5.0 fold, or 6-fold.

In one embodiment of the invention, the differential expression of the at least two, and up to 26, biomarkers in the test sample is determined to exceed that in the control sample by more than the 3-fold, 3.5-fold, or 4.0 fold.

In one embodiment of the invention, the differential expression of the at least two, and up to 24, biomarkers in the test sample is determined to be reduced relative to that in the control sample by more than the 3-fold, 3.5-fold, 4-fold, 5-fold, or 6.0 fold.

In a specific embodiment of the invention, the differential expression of the at least two, and
(a) up to 19, biomarkers selected from the group of biomarkers depicted in Table 1 identified by GeneBank Accession number A1634580, NM_001611, NM_000669, D26054, AA551075, BG546884, NM_023504, Al382123, NM_002656, AW500340, Al694562, NM_000550. AA858297, BC001606, NM_001629, BE881590, AI680192, AW027333, and BF446673; or
(b) up to 13, biomarkers selected from the group of biomarkers depicted in Table 2 identified by GeneBank Accession number NM_001657, NM_001565, AA496034, M87789, BG536224, NM_018407, W46388, S59049, AU146532, L24521, NM_000224, AW149681, and NM_002627; or
(c) up to 32, biomarkers selected from the group of biomarkers depicted in Table 1 and Table 2 identified by GeneBank Accession number AI634580, NM_001611, NM_000669, D26054, AA551075, BG546884, NM_023504, AI382123, NM_002656, AW500340, AI694562, NM_000550. AA858297, BC001606, NM_001629, BE881590, AI680192, AW027333, BF446673, NM_001657, NM_001565, AA496034, M87789, BG536224, NM_018407, W46388, S59049, AU146532, L24521, NM_000224, AW149681, and NM_002627,

in the test sample is determined to be reduced relative to that in the control sample by more than the 3-fold, 3.5-fold, 4-fold, 5-fold, or 6.0 fold.

In one embodiment of the invention, said test sample is obtained from an individual, who is a smoker or a non-smoker and the control sample can be one or more of the following types of tissue:
a. Adenocarcinoma-smoker-normal tissue;
b. Adenocarcinoma-smoker-tumor tissue;
c. Adenocarcinoma-non-smoker-normal tissue;
d. Adenocarcinoma-non-smoker-tumor tissue;
e. Benign-non-smoker-tumor tissue; and
f. Benign- smoker-tumor tissue.

In particular, the present invention relates to a method for determining in an individual lung cancer or a predisposition for lung cancer said method comprising
i. determining in a biological sample of an individual suspected of suffering from lung cancer or of being predisposed for lung cancer, a target value for a panel of at least 2 and up to 50 different target biomarkers selected from the biomarkers depicted in Table 1, Table 2, or both Table 1 and Table 2;
ii. determining a reference value for said target biomarkers in a non-cancerous control sample;
iii. comparing the target value to the reference value; and
iv. predicting whether or not said individual has or is predisposed for lung cancer.

In one embodiment, the invention provides a method of distinguishing and classifying normal and tumor tissues comprising determining in said tissues differential expression of a panel of at least 5, particularly at least 6, particularly at least 7, particularly at least 8, particularly at least 9, particularly at least 10 biomarkers, selected from the biomarkers depicted in Table 1, Table 2, or both Table 1 and Table 2.

In particular, said method comprises
i. determining in a tissue sample of an individual suspected of suffering from lung cancer a target value for the panel of target biomarkers as mentioned above;
ii. determining a reference value for said target biomarkers in a non-cancerous control sample;
iii. comparing the target value to the reference value; and
iv. classifying the sample tissue as normal or tumor tissue.

In one embodiment, the invention provides a method of distinguishing and classifying normal and tumor tissues and further discriminating between patients based on smoking status, comprising determining in said tissues differential expression of a panel of at least 25, particularly at least 40, particularly at least 50 biomarkers, selected from the biomarkers depicted in Table 1, Table 2, or both Table 1 and Table 2.

In particular, said method comprises
i. determining in a tissue sample of an individual suspected of suffering from lung cancer a target value for the panel of target biomarkers as mentioned above;
ii. determining a reference value for said target biomarkers in a non-cancerous control sample;
iii. comparing the target value to the reference value; and
iv. determining the smoking status of the individual and classifying the sample tissue as normal or tumor tissue.

In one embodiment of the invention, the reference value for said target biomarkers are determined based on one or more of the following types of tissues:
a. Adenocarcinoma-smoker-normal tissue;
b. Adenocarcinoma-smoker-tumor tissue
c. Adenocarcinoma-non-smoker-normal tissue;
d. Adenocarcinoma-non-smoker-tumor tissue;
e. Benign-non-smoker; and
f. Benign- smoker.

In one embodiment, the present invention provides a method for monitoring the progress of a lung cancer treatment in an individual, said method comprising determining at suitable time intervals before, during, or after lung cancer therapy, particularly at different time points during the treatment, in a sample taken from said individual differential expression of a panel of at least 2 and up to 50 different biomarkers selected from the biomarkers depicted in Table 1, Table 2, or both Table 1 and Table 2.

In particular, said method comprises
i. determining at various time points in a biological sample of an individual treated for lung cancer with a curative intent a target value for a panel of at least 2 and up to 50 different target biomarkers selected from the biomarkers depicted in Table 1, Table 2, or both Table 1 and Table 2;
ii. comparing the target value to a reference value determined in a non-cancerous control sample at the beginning of the treatment; and
iii. determining the progress made.

In one embodiment, the sample is blood, serum, plasma, sputum, saliva, tissue particularly lung tissue, obtained through biopsy, bronchia brushings, exhaled breath, or urine.

In a specific embodiment of the invention, the target value in a biological sample is determined within any of the methods of the invention as described herein in the various embodiments for a panel of at least 2 and up to 50 different biomarkers
(a) depicted in Table 1 selected from the group of biomarkers identified by GeneBank Accession number AI634580, NM_001611, NM_000669, D26054, AA551075, BG546884, NM_023504, AI382123, NM_002656, AW500340, AI694562, NM_000550. AA858297, BC001606, NM_001629, BE881590, AI680192, AW027333, and BF446673; or
(b) depicted in Table 2 selected from the group of biomarkers identified by GeneBank Accession number NM_001657, NM_001565, AA496034, M87789, BG536224, NM_018407, W46388, S59049, AU146532, L24521, NM_000224, AW149681, and NM_002627; or
(c) depicted in Table 1 and Table 2 selected from the group of biomarkers identified by GeneBank Accession number AI634580, NM_001611, NM_000669, D26054, AA551075, BG546884, NM_023504, AI382123, NM_002656, AW500340, AI694562, NM_000550. AA858297, BC001606, NM_001629, BE881590, AI680192, AW027333, BF446673, NM_001657, NM_001565, AA496034, M87789, BG536224, NM_018407, W46388, S59049, AU146532, L24521, NM_000224, AW149681, and NM_002627.

In one embodiment of the invention, determination of biomarker values is accomplished by performing an *in-vitro* assay, particularly an *in-vitro* assay selected from the group consisting of an antibody-based assay such as an immunoassay, a histological or cytological assay, an expression level assay such as an RNA expression level assay and an aptamer-based assay.

In one embodiment of the invention, the biomarker value is determined by performing mass spectrometry.

In a specific embodiment of the invention, an enzyme-linked immunosorbent assay may be performed for determining biomarker values.

In another specific embodiment of the invention, a microarray-based immunohistochemical analysis may be performed for determining biomarker values.

In still another specific embodiment of the invention, surface enhanced laser desorption/ionization may be performed for determining biomarker values.

In one aspect of the invention, data analysis is performed within a method according to any one of the preceding embodiments by one or more computer program(s).

In one embodiment, the present invention provides a panel of at least 2, at least 3, at least 4, at least 5, at least 6, at least 7, at least 8, at least 9, at least 10, at least 15, at least 20, at least 25, at least 30, at least 35, at least 40, at least 45, at least 50 biomarkers selected from the group of markers provided in Table 1, Table 2, or both Table 1 and Table 2, for use in a method according to any one of the preceding embodiments.

In a specific embodiment, a panel of at least 10 biomarkers is provided selected from the group of markers depicted in Table 1, Table 2, or both Table 1 and Table 2 for use in a method according to any one of the preceding embodiments.

In another specific embodiment of the invention, a panel of at least 50 biomarkers is provided selected from the group of markers depicted in Table 1, Table 2, or both

Table 1 and Table 2 for use in a method according to any one of the preceding embodiments.

In one embodiment, the present invention provides a kit for determining in an individual lung cancer or a predisposition for lung cancer or for monitoring the progress of a lung cancer treatment in an individual, comprising a reagent for detecting differential expression of a panel of at least 2 and up to 50 different biomarkers selected from the biomarkers depicted in Table 1, Table 2, or both Table 1 and Table 2.

In a specific aspect, the present invention relates to a panel of biomarkers selected from the biomarkers depicted in Table 1, Table 2, or both Table 1 and Table 2 comprising those biomarkers from said tables which are identified by GeneBank Accession identifiers and to the use of said biomarkers or panel of biomarkers in a method or a kit according to the invention and described herein in the various embodiments.

In particular, the present invention relates to a panel of biomarkers
(a) depicted in Table 1 selected from the group of biomarkers identified by GeneBank Accession number AI634580, NM_001611, NM_000669, D26054, AA551075, BG546884, NM_023504, AI382123, NM_002656, AW500340, AI694552, NM_000550. AA858297, BC001606, NM_001629, BE881590, AI680192, AW027333, and BF446673; or
(b) depicted in Table 2 selected from the group of biomarkers identified by GeneBank Accession number NM_001657, NM_001565, AA496034, M87789, BG536224, NM_018407, W46388, S59049, AU146532, L24521, NM_000224, AW149681, and NM_002627; or
(c) depicted in Table 1 and Table 2 selected from the group of biomarkers identified by GeneBank Accession number AI634580, NM_001611, NM_000669, D26054, AA551075, BG546884, NM_023504, AI382123, NM_002656, AW500340, AI694562, NM_000550. AA858297, BC001606, NM_001629, BE881590, AI680192, AW027333, BF446673, NM_001657, NM_001565, AA496034, M87789, BG536224, NM_018407, W46388, S59049, AU146532, L24521, NM_000224, AW149681, and NM_002627.

The present invention provides biomarkers or a combination of biomarkers that are predictive for smoke-induced lung cancer development and that can be used to develop tools in order to decrease adverse health effects caused by exposure to tobacco smoke and other tobacco-related products.

In one aspect of the present invention, biomarkers (genes) were identified based on gene expression patterns that can detect smoke-induced lung cancer development. In another aspect, the present invention provides biomarkers that can be used to distinguish between pre-stages or early stages of lung cancer and advanced stages of lung cancer ("early predictive markers").

According to the invention, a high level of expression of a target biomarker relative to a reference level indicates a prognosis or diagnosis of lung caner; or a low level of expression of a target biomarker relative to a reference level indicates a lower risk of lung cancer. The reference level of a biomarker can be established from cells from characterized cell lines, or cells from a clinically-characterized population of patients, such as, but not limited to, patients that have the aggressive form of the disease, patients that have the disease in remission, smokers that have an adenocarcinoma; non-smokers who have an adenocarcinoma; smokers who have never had lung cancer, or non-smokers who have never had lung cancer.

The method of the invention comprises measuring at suitable time intervals before, during, or after lung cancer therapy, the amount of biomarker gene product. Any change or absence of change in the amount of the biomarker gene product can be identified and correlated with the effect of the treatment on the subject. In a preferred aspect, the method comprises determining the levels of biomarker gene product levels at different time points and to compare these values with a reference level. The reference level can be either the level of the biomarker present in normal, disease free individuals, individuals with characterized disease (aggressive disease or staged disease); and/or the levels present prior to treatment, or during remission of disease, or during periods of stability. These levels can then be correlated with the disease course, treatment outcome or overall survival.

Detection of the protein biomarkers described herein in a test sample may be performed in a variety of ways well known to those skilled in the art.

In one aspect, the methods of the invention rely on the detection of the presence or absence of biomarker gene expression, or the qualitative or quantitative assessment of either over- or under-expression of biomarker gene in a population of test cells relative to a standard. Such methods utilize reagents such as biomarker polynucleotides and biomarker antibodies as described herein.

In particular, the level of expression of a biomarker gene may be determined by measuring the amount of biomarker messenger RNA, for example, by DNA-DNA hybridization, RNA-DNA hybridization, reverse transcription-polymerase chain reaction (PCR), or real time quantitative PCR; followed by comparing the results to a reference based on samples from clinically-characterized patients and/or cell lines of a known genotype/phenotype. As an alternative to amplification techniques, hybridization assays can be performed.

For example, these techniques find application in microarray-based assays that can be used to detect and quantify the amount of biomarker gene transcript using cDNA- or oligonucleotide-based arrays. Microarray technology allows multiple biomarker gene transcripts and/or samples from different subjects to be analyzed in one reaction. Typically, mRNA isolated from a sample is converted into labeled nucleic acids by reverse transcription and optionally in vitro transcription (cDNAs or cRNAs labeled with, for example, Cy3 or Cy5 dyes) and hybridized in parallel to probes present on an array. See, for example, Schulze et al., Nature Cell Biol., 3 (2001), E190; and Klein et al., J Exp Med, 2001, 1625-1638, which are incorporated herein by reference in their entirety. Standard Northern analyses can be performed if a sufficient quantity of the test cells can be obtained. Utilizing such techniques, quantitative as well as size related differences between biomarker transcripts can also be detected.

The present invention provides isolated biomarker polynucleotides or variants thereof, which can be used, for example, as hybridization probes or primers ("biomarker probes" or "biomarker primers") to detect or amplify nucleic acids encoding a biomarker polypeptide, particularly a biomarker polypeptide encoded by a biomarker gene or polynucleotide selected from the group depicted in Table 1, Table 2, or both Table 1 and Table 2.

Nucleic acid molecules comprising nucleic acid sequences encoding the biomarker polypeptides or proteins of the invention, or genomic nucleic acid sequences from the biomarker genes (e.g., intron sequences, 5' and 3' untranslated sequences), or their complements thereof (i.e., antisense polynucleotides), are collectively referred to as "biomarker genes", "biomarker polynucleotides" or "biomarker nucleic acid sequences" of the invention. The present invention also provides isolated biomarker polynucleotides or variants thereof, which can be used, for example, as hybridization probes or primers ("biomarker probes" or "biomarker primers") to detect or amplify nucleic acids encoding a polypeptide of the invention. The term "biomarker gene product" thus encompasses both mRNA as well as translated polypeptide as a gene product of a biomarker.

The isolated biomarker polynucleotide according to the invention may comprise flanking sequences (*i.e*., sequences located at the 5' or 3' ends of the nucleic acid), which naturally flank the nucleic acid sequence in the genomic DNA of the organism from which the nucleic acid is derived. However, an isolated polynucleotide does not include an isolated chromosome, and does not include the poly(A) tail of an mRNA, if present. For example, in various embodiments, the isolated biomarker polynucleotide can comprise less than about 5 kb, 4 kb, 3 kb, 2 kb, 1 kb, 0.5 kb or 0.1 kb of nucleotide sequences which naturally flank the coding sequence in genomic DNA of the cell from which the nucleic acid is derived. In other embodiments, the isolated biomarker polynucleotide is about 10-20, 21-50, 51-100, 101-200, 201-400, 401-750, 751-1000, 1001-1500 bases in length.

In various embodiments, the biomarker polynucleotides of the invention are used as molecular probes in hybridization reactions or as molecular primers in nucleic acid extension reactions as described herein. In these instances, the biomarker polynucleotides may be referred to as biomarker probes and biomarker primers, respectively, and the biomarker polynucleotides present in a sample which are to be detected and/or quantified are referred to as target biomarker polynucleotides. Two biomarker primers are commonly used in DNA amplification reactions and they are referred to as biomarker forward primer and biomarker reverse primer depending on their 5' to 3' orientation relative to the direction of transcription. A biomarker probe or a biomarker primer is typically an oligonucleotide which binds through complementary base pairing to a subsequence of a target biomarker polynucleotide. The biomarker probe may be, for example, a DNA fragment prepared by amplification methods such as by PCR or it may be chemically synthesized. A double stranded fragment may then be obtained, if desired, by annealing the chemically synthesized single strands together under appropriate conditions or by synthesizing the complementary strand using DNA polymerase with an appropriate primer. Where a specific nucleic acid sequence is given, it is understood that the complementary strand is also identified and included as the complementary strand will work equally well in situations where the target is a double stranded nucleic acid. A nucleic acid probe is complementary to a target nucleic acid when it will anneal only to a single desired position on that target nucleic acid under proper annealing conditions which depend, for example, upon a probe's length, base composition, and the number of mismatches and their position on the probe, and must often be determined empirically. Such conditions can be determined by those of skill in the art.

In one aspect of the invention, biomarkers may be detected in the test sample by gene expression profiling. In these methods, mRNA is prepared from a sample and mRNA expression levels are measured by reverse transcription quantitative polymerase chain reaction (RT-PCR followed with qPCR). RT-PCR is used to create a cDNA from the corresponding mRNA. The cDNA may be used in a qPCR assay to produce fluorescence as the DNA amplification process progresses. By comparison to a standard curve, qPCR can produce an absolute measurement such as number of copies of mRNA per cell. Northern blots, microarrays, Invader assays, and RT-PCR combined with capillary electrophoresis may be used to measure expression levels of mRNA in a sample. Further details are provided, for example, in "Gene Expression Profiling: Methods and Protocols", Richard A. Shimkets, editor, Humana Press, 2004 and US patent application 20100070191.

In another aspect of the invention, detection of the biomarkers described herein may also be accomplished by an immunoassay procedure. The immunoassay typically includes contacting a test sample with an antibody that specifically binds to or otherwise recognizes a biomarker, and detecting the presence of the antibody/biomarker complex in the sample. The immunoassay procedure may be selected from a wide variety of immunoassay procedures known to those skilled in the art such as, for example, competitive or non-competitive enzyme-based immunoassays, enzyme-linked immunosorbent assays (ELISA), radioimmunoassay (RIA), and Western blots, etc. Further, multiplex assays may be used, including antibody arrays, wherein several desired antibodies are placed on a support, such as a glass bead or plate, and reacted or otherwise contacted with the test sample. Antibodies used in these assays may be monoclonal or polyclonal, and may be of any type such as IgG, IgM, IgA, IgD and IgE. Monoclonal antibodies may be used to bind to a specific epitope offered by the biomarker molecule, and therefore may provide a more specific and accurate result. Antibodies may be produced by immunizing animals such as rats, mice, and rabbits. The antigen used for immunization may be isolated from the samples or synthesized by recombinant protein technology. Methods of producing antibodies and of performing antibody-based assays are well-known to the skilled artisan and are described, for example, more thoroughly in Antibodies: A Laboratory Manual (1988) by Harlow & Lane; Immunoassays: A Practical Approach, Oxford University Press, Gosling, J. P. (ed.) (2001) and/or Current Protocols in Molecular Biology (Ausubel et al.) which is regularly and periodically updated.

In certain embodiments, the present invention also provides "biomarker antibodies" including polyclonal, monoclonal, or recombinant antibodies, and fragments and variants thereof, that immunospecifically binds the respective biomarker proteins or polypeptides encoded by the genes or cDNAs (including polypeptides encoded by mRNA splice variants) as listed in Tables 1 and 2..

Various chemical or biochemical derivatives of the antibodies or antibody fragments of the present invention can be produced using known methods. One type of derivative which is diagnostically useful as an immunoconjugate comprising an antibody molecule, or an antigen-binding fragment thereof, to which is conjugated a detectable label. However, in many embodiments, the biomarker antibody is not labeled but in the course of an assay, it becomes indirectly labeled by binding to or being bound by another molecule that is labeled. The invention encompasses molecular complexes comprising a biomarker antibody and a label, as well as immunocomplexes comprising a biomarker polypeptide, a biomarker antibody, and immunocomplexes comprising a biomarker polypeptide, a biomarker antibody, and a label.

Examples of detectable substances include various enzymes, prosthetic groups, fluorescent materials, luminescent materials, bioluminescent materials, and radioactive materials. Examples of suitable enzymes include horseradish peroxidase, alkaline phosphatase, β-galactosidase, or acetylcholinesterase; examples of suitable prosthetic group complexes include streptavidin/biotin and avidin/biotin; examples of suitable fluorescent materials include umbelliferones, fluoresceins, fluorescein isothiocyanate, rhodamines, dichlorotriazinylamine fluorescein, dansyl chloride, phycoerythrins, Alexa Fluor 647, Alexa Fluor 680, DiIC₁₉(3), Rhodamine Red-X, Alexa Fluor 660, Alexa Fluor 546, Texas Red, YOYO-1 + DNA, tetramethylrhodamine, Alexa Fluor 594, BODIPY FL, Alexa Fluor 488, Fluorescein, BODIPY TR, BODIPY TMR, carboxy SNARF-1, FM 1-43, Fura-2, Indo-1, Cascade Blue, NBD, DAPI, Alexa Fluor 350, aminomethylcoumarin, Lucifer yellow, Propidium iodide, or dansylamide; an example of a luminescent material includes luminol; examples of bioluminescent materials include green fluorescent proteins, modified green fluorescent proteins, luciferase, luciferin, and aequorin, and examples of suitable radioactive material include 125_{I} 131_{I} 35_{S} or 3_{H}.

Immunoassays for biomarker polypeptides will typically comprise incubating a sample, such as a biological fluid, a tissue extract, freshly harvested cells, or lysates of cells, in the presence of a detectably labeled antibody capable of identifying biomarker gene products or conserved variants or peptide fragments thereof, and detecting the bound antibody by any of a number of techniques well-known in the art. One way of measuring the level of biomarker polypeptide with a specific biomarker antibody of the present invention is by enzyme immunoassay (EIA) such as an enzyme-linked immunosorbent assay (ELISA) (Voller, A. et al., J. Clin. Pathol. 31:507-520 (1978); Butler, J.E., Meth. Enzymol. 73:482-523 (1981); Maggio, E. (ed.), Enzyme Immunoassay, CRC Press, Boca Raton, FL, 1980). The enzyme, either conjugated to the antibody or to a binding partner for the antibody, when later exposed to an appropriate substrate, will react with the substrate in such a manner as to produce a chemical moiety which can be detected, for example, by spectrophotometric, or fluorimetric means.

The biological sample may be brought in contact with and immobilized onto a solid phase support or carrier such as nitrocellulose, or other solid support which is capable of immobilizing cells, cell particles or soluble proteins. The support may then be washed with suitable buffers followed by treatment with the detectably labeled biomarker antibody. The solid phase support may then be washed with the buffer a second time to remove unbound antibody. The amount of bound label on solid support may then be detected by conventional means. A well known example of such a technique is Western blotting.

In various embodiments, the present invention provides compositions comprising labeled biomarker polynucleotides, or labeled biomarker antibodies to the biomarker proteins or polypeptides or labeled biomarker polynucleotides and labeled biomarker antibodies to the biomarker proteins or polypeptides according to the invention as described herein..

In addition to antibody-based techniques, the biomarkers described herein may also be detected and quantified by mass spectrometry. Mass spectrometry is a method that employs a mass spectrometer to detect ionized protein markers or ionized peptides as digested from the protein markers by measuring the mass-to-charge ratio (m/z). Labeling of biomarkers (along with other proteins) with stable heavy isotopes (deuterium, carbon-13, nitrogen-15, and oxygen-18) can be used in quantitative proteomics. These are either incorporated metabolically in sample cells cultured briefly in vitro, or directly in samples by chemical or enzymatic reactions. Light, and heavy labeled biomarker peptide ions segregate and their intensity values are used for quantification. For example, analytes may be introduced into an inlet system of the mass spectrometer and ionized in an ionization source, such as a laser, fast atom bombardment, plasma or other suitable ionization sources known to the art. The generated ions are typically collected by an ion optic assembly and introduced into mass analyzers for mass separation before their masses are measured by a detector. The detector then translates information obtained from the detected ions into mass-to-charge ratios.

The invention also provides compositions comprising biomarker polynucleotides, biomarker polypeptides, or biomarker antibodies according to the invention as described herein in the various embodiments. The invention further provides diagnostic reagents for use in the methods of the invention, such as but not limited to reagents for flow cytometry and/or immunoassays that comprise fluorochrome-labeled antibodies that bind to one of the biomarker polypeptides of the invention as described herein.

In one embodiment, the invention provides diagnostic reagents that comprise one or more biomarker probes, or one or more biomarker primers. A diagnostic reagent may comprise biomarker probes and/or biomarker primers from the same biomarker gene or from multiple biomarker genes. In another embodiment, the invention also provides diagnostic compositions that comprise one or more biomarker probes and target biomarker polynucleotides, or one or more biomarker primers and target polynucleotides, or biomarker primers, biomarker probes and biomarker target polynucleotides. In some embodiments, the diagnostic compositions comprise biomarker probes and/or biomarker primers and a sample suspected to comprise biomarker target polynucleotides. Such diagnostic compositions comprise biomarker probes and/or biomarker primers and the nucleic acid molecules (including RNA, mRNA, cRNA, cDNA, and/or genomic DNA) of a subject in need of a diagnosis/prognosis of lung cancer.

The present invention also provides kits for practicing the methods of the invention. The kits can be used for clinical diagnosis and/or laboratory research. In one embodiment, a kit comprises one or more diagnostic reagents in one or more containers. Preferably, the kit also comprises instructions in any tangible medium on the use of the diagnostic reagent(s) in one or more methods of the invention.

For nucleic acid-based methods, such as hybridization assays or polymerase chain reaction, a diagnostic reagent in the kit may comprise at least one biomarker polynucleotide, biomarker probe, and/or biomarker primer based on the biomarkers depicted in Table 1, Table 2, or both Table 1 and Table 2. The diagnostic reagents may be labeled, for example, by one or more different fluorochromes. Such a kit may optionally provide in separate containers enzymes and/or buffers for reverse transcription, in vitro transcription, and/or DNA polymerization, nucleotides, and/or labeled nucleotides, including fluorochrome-labeled nucleotides. Also included in the kit may be positive and negative controls for the methods of the invention.

For protein-based methods, such as immunoassays, a diagnostic reagent in the kit may comprise a biomarker antibody, which may be labeled, for example, by a fluorochrome. Such a kit may optionally provide in separate containers buffers, secondary antibodies, signal generating accessory molecules, labeled secondary antibodies, including fluorochrome-labeled secondary antibodies. The kit may also include unlabeled or labeled antibodies to various cell surface antigens which can used for identification or sorting of subpopulations of cells. Also included in the kit may be positive and negative controls for the methods of the invention. The positive and/or negative controls included in a kit can be nucleic acids, polypeptides, cell lysate, cell extract, whole cells from patients, or whole cells from cell lines.

The foregoing description will be more fully understood with the reference to the following Examples. Such Examples are, however, exemplary methods of practicing the present invention and are not intended to limit the scope of the invention.

### EXAMPLES

In order to identify biomarkers that can be used to distinguish between pre-stages or early stages of lung cancer and advanced stages of lung cancer ("early predictive markers"), in a first study (study 1; "training set") a total of 90 lung tissue samples from "healthy" donors and from lung cancer patients with focus on adenocarcinomas were collected and analyzed by an Affymetrix gene expression analysis. All patients were grouped according to their tumor, smoking and tissue status and matched based on their general anamnesis information (e.g. age, gender) and selected clinical chemistry data. The analysis of the expression data of the first study led to the identification of 578 genes (838 genes) that were more than 3-fold differently expressed (mean signal values with p< 0.05) between "normal" tissues and matched tumor tissues (cancer patients) in non-smokers (smokers) as well as 620 genes (860 genes) that were differently expressed (mean signal values with p< 0.05) between normal control tissues (control patients) and tumor tissues (cancer patients) in non-smokers (smokers). Furthermore 50 biomarker candidates (genes) were identified as being specific for smokers which showed a more than 3-fold expression difference between normal and lung tumor tissue and were reliably expressed in 95 % of the arrays.

Subsequently a second study (study 2;"testing set") with an additional set of 90 independent tissue samples from 60 different patients was performed which substantiated the results obtained in study 1.

### 1. Basic data preparation and evaluation

The quality control and data analysis steps were performed using various software packages (GCOS 1.3 and Quality data reporter/Affymetrix; Array Assist 4.1/Stratagene; Statistica/Statsoft). The obtained .cel files were generated by scanning the arrays and represent the raw data (signal intensity for each probe cell) for each chip (about 1.3 Mio data points per array). They were used as basis for all subsequent analysis procedures. Signal intensities and gene detection calls were calculated and derived from the .cel files using Affymetrix algorithm MAS 5.0 according to Affymetrix software standard procedure (Affymetrix Microarray suite/GCOS 1.3 version software). The resulting single weighted mean signal intensities, which assign a relative measure of abundance for each probe set on each array, are listed in the chp files and are also exported into Excel as .xls files.

### 2. Exploratory data analysis of gene signatures ― Statistical analysis of group differences.

All arrays (N=90) with a total of 54,674 probe sets per array were included in the analysis. The arrays were first grouped for statistical examinations and comparisons according to their status (tumor, tissue type, smoker/non-smoker):
Patient status:
   - AS-N (Adenocarcinoma-smoker-normal tissue)
   - AS-T (Adenocarcinoma-smoker-tumor tissue)
   - ANS-N (Adenocarcinoma-non smoker-normal tissue)
   - ANS-T (Adenocarcinoma-non smoker-tumor tissue)
   - BNS (Benign-non smoker)
   - BS (Benign-smoker)

To check for data quality and to examine the arrays for underlying data structures, as a next step Principal Component Analysis (PCA, mean centered, factor number limited to 4 [E0 to E3], Array assist software) was performed. For this analysis method the signal intensities values of all gene probe sets (.chp files) were used in order to view potential clusters of patient group replicates or to identify separations between groups depending on selected grouping parameters. The PCA analysis in general showed no distinct clustering of the arrays based on smoking Status. In contrast, a clear clustering and separation based on tissue status was observed with a separation between the normal (N) and tumor tissues (T), in the plane of the two main components demonstrating an underlying separating structure in the data sets. However, no further clustering was observed for either biological replicates within the different patient groups, for smoking status, whether the normal samples were derived from metastatic cancer patients (ICD code 78) or on the basis of tumor proportion within the tumor patient samples.

Hierarchical cluster analysis for array similarities based on all probe set signals was performed. Unsupervised cluster analysis was based on Eucledian distance calculations and complete linkage (maximum distance) of the samples to identify similarities and segments between the arrays. The analysis yielded a similar separation pattern as obtained using the PCA analysis approach. Arrays hybridized with tumor tissue RNAs were clearly distinguished from arrays exposed to normal tissue RNAs and formed one main cluster, whereas all normal tissue arrays formed a second cluster.

To further examine the relationships between the arrays we also performed a correlation analysis with the entire array set. This analysis underscored the PCA and cluster analysis and demonstrated a high correlation within the tumor arrays and within normal tissue arrays and low correlation between the two groups.

To prepare the data sets for probe set expression analysis we first extracted probe sets which were not reliably detected prior to statistical data analysis by filtering for uninformative absent calls within the entire array set of the experimental groups. Probe sets classified as absent in all arrays were excluded from the subsequent analysis (a total of 13630 probe sets were excluded this way when this filter step was applied to all 90 arrays leaving a total of 41045 probe sets for further analysis). Before the gene signal values were log2 transformed to analyse gene expression differences between the groups, a variance stabilization step was performed by adding the artificial value of 16 (Array assist software default value) to all values to suppress noise at log signal values.

### 3. Group to group comparisons

Advanced statistical analysis was then performed using the log2 transformed values to generate gene lists for differentially expressed probe sets between the various experimental groups. Unpaired tests were performed to compare patient, tumor, tissue and smoking status. Paired tests were used to examine normal vs. tumor tissue within the AS and ANS groups (i.e. AS-N vs. AS-T and ANS-N vs. ANS-T comparisons). Significance tests for probe set expression differences between two groups were performed using t-test or Mann-Whitney test. Comparisons between multiple groups were done using the Kruskal Wallis test.

P values were computed using an asymptotic approach assuming a normal distribution of the p values. Corrections of the p values for multiple testing were either not applied to yield an estimate of the maximum number of differently expressed genes or the Benjamini-Hochberg algorithm was used for adjustment of p values to correct for false positive discovery rates.

The threshold value for significance was set to p < 0.05 for all statistical analysis methods.

Initially, the focus was on gene expression differences larger or equal three to identify genes with robust changes.. The resulting gene lists with the significant > 3 fold changes of expression levels were routinely obtained as intersections of t- and Mann Whitney tested gene probe lists with asymptotic p values corrected with the Benjamini Hochberg algorithm. The gene list were then subsequently analysed in serial Venn diagrams for intersection or exclusion to specific transcriptomes and subgroups. The following comparisons were made:

### 3.1 Group to group comparisons non smokers:

- Normal tissue lung cancer patients vs. tumor tissue lung cancer patients. (ANS-N vs. ANS-T, paired analysis)
- Normal tissue benign lung disease patients vs. tumor tissue lung cancer patients. (BNS vs. ANS-T, unpaired analysis)
- Normal tissue benign lung disease patients vs. normal tissue from lung cancer patients. (BNS vs. ANS-N, unpaired analysis)

### 3.2 Group to group comparisons smokers:

- Normal tissue lung cancer patients vs. tumor tissue lung cancer patients. (AS-N vs. AS-T, paired analysis)
- Normal tissue benign lung disease patients vs. tumor tissue lung cancer patients. (BS vs. AS-T, unpaired analysis)
- Normal tissue benign lung disease patients vs. normal tissue from lung cancer patients. (BS vs. AS-N, unpaired analysis)

### 3.3 Group to group comparison non smokers vs. smokers:

- Gene probes only differentially expressed in non smokers group BNS vs. ANS-T (group A) against gene probes only differentially expressed in smokers BS vs. AS-T (group B)

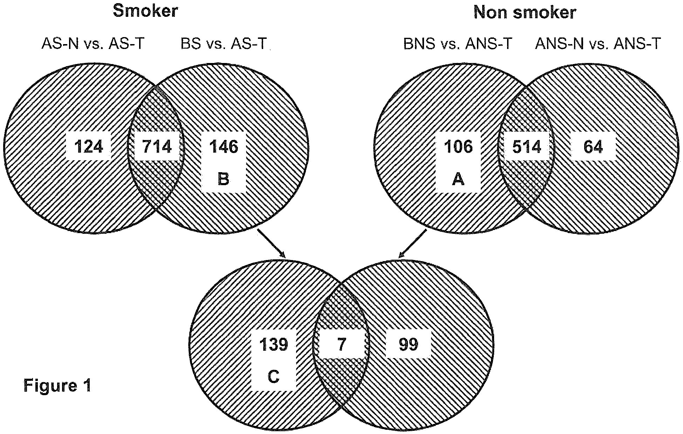

Serial Venn diagram analysis of gene probes with changed expression levels of both remaining non smoking groups (BNS vs. ANS-T and ANS-N vs. ANS-T) revealed 106 gene probe sets which were only changed between the BNS vs. ANS-T group, 514 identical gene probes changing simultaneously in both non smoking groups and 64 gene probes which were specifically changed between the ANS-N and ANS-T samples. The 106 gene probes were designated as group A. Applying the same examination of the two remaining smoking groups yielded 146 gene probe sets which were only changed between the BS and AS-T group, 714 identical sets that were simultaneously altered in both smoking groups and 124 gene probes specifically changed between the AS-N and AS-T group. The obtained 146 gene probe set was designated as group B. To yield potentially smoking induced candidate genes the group A and B gene probes sets were further examined in a Venn diagram. The gene probes expressed only in the smoking subset were selected as potential smoking induced gene markers and designated as group C. The group C contains 139 gene probes that are possible candidates for smoke induced cancer growth. These genes are largely identical with the group B gene set of which only 7 genes were found in the non-smoker group. When we focused our analysis on genes that were expressed by the majority of patients probes (> 88 of arrays) and, thus, provided a clear signal intensity for quantification. Overall 50 expressed genes among the 139 genes were identified that are of particular interest for further evaluation as smoke-induced biomarker. One gene - CRIM1 were counted twice in the table as different probe sets were used in the analysis. Figure 1 shows the Venn diagram analysis to identify potential early tumor marker gene probes.

Genes identified in group C which are specifically upregulated in smoking tumor patients (AS-T) versus smoking control patients (BS) are listed in Table 1 Table 2 contains the list of genes which are down-regulated in the AS-T group compared to the BS group. The absolute fold changes (FC) and the respective p values are summarized in columns 4 and 5.

In total 50 genes were identified satisfying the filter criteria.

**Table 1: Genes which are specifically upregulated in smoking tumor patients (AS-T) compared to smoking control patients (BS)**

| **A)** | | | | | |
|---|---|---|---|---|---|
| **Probe Set ID*** | **Gene Symbol** | **Regulation ([AS-T] vs [BS])** | **FC Absolute ([AS-T] vs [BS])** | **P-value ([AS-T] vs [BS])** | **GeneBank Accession** |
| 1555745_a_ at | LYZ | up | 4.3 | 1.21E-03 | --- |
| 225895_at | SYNPO2 | up | 3.6 | 4.77E-08 | AI634580 |
| 204638_at | ACP5 | up | 3.5 | 3.56E-08 | NM_001611 |
| 206262_at | ADH1A | up | 3.4 | 3.40E-05 | NM_000669 |
| 209696_at | FBP1 | up | 3.4 | 1.51 E-05 | D26054 |
| 212188_at | KCTD12 | up | 3.3 | 3.91 E-09 | AA551075 |
| 202551_s_at | CRIM1 | up | 3.3 | 4.26E-07 | BG546884 |
| 228731_at | AK001875 | up | 3.3 | 1.06E-06 | NM_023504 |
| 217177_s_at | PTPRB | up | 3.3 | 7.34E-12 | ---- |
| 213067_at | MYH10 | up | 3.2 | 8.32E-08 | AI382123 |
| 207002_s_at | PLAGL1 | up | 3.2 | 1.96E-06 | NM 002656 |
| 212451_at | KIAA0256 | up | 3.1 | 1.97E-10 | ---- |
| 228496_s_at | CRIM1 | up | 3.1 | 5.52E-07 | ---- |
| 216012_at | U43604 | up | 3.1 | 1.13E-06 | ---- |
| 242714_at | Hs.672896 | up | 3.1 | 5.05E-08 | AW500340 |
| 222073_at | COL4A3 | up | 3.1 | 7.91 E-04 | AI694562 |
| 205694_at | TYRP1 | up | 3.1 | 1.94E-09 | NM 000550 |
| 228071_at | GIMAP7 | up | 3.1 | 6.11E-07 | AA858297 |
| 209949_at | NCF2 | up | 3.1 | 3.12E-07 | BC001606 |
| 204174_at | ALOX5AP | up | 3.1 | 1.94.E-09 | NM_001629 |
| 221911_at | ETV1 | up | 3.1 | 5.99E-07 | BE881590 |
| 1553243_at | ITIH5 | up | 3.0 | 1.43E-07 | ---- |
| 212239_at | PIK3R1 | up | 3.0 | 7.42E-11 | AI680192 |
| 225782_at | MSRB3 | up | 3.0 | 4.45E-07 | AW027333 |
| 238363_at | CAT | up | 3.0 | 3.54E-09 | ---- |
| 235944_at | HMCN1 | up | 3.0 | 9.75E-07 | BF446673 |

**Table 2: Genes which are down regulated in the smoking tumor patients (AS-T) compared to smoking control patients (BS)**

| **B)** | | | | | |
|---|---|---|---|---|---|
| **Probe Set ID*** | **Gene Symbol** | **Regulation ([AS-T] vs [BS])** | **FC Absolute ([AS-T] vs [BS])** | **P-value ([AS-T] vs [BS])** | **GeneBank Accession** |
| 215946_x_ at | LOC91353 | down | 6.7 | 2.92E-06 | ---- |
| 234764_x_at | IGLC2 | down | 6.3 | 4.10E-05 | ---- |
| 217480_x_at | LOC339562 | down | 6.0 | 2.33E-05 | ---- |
| 215176_x_at | IGKC | down | 5.0 | 4.23E-06 | ---- |
| 215214_at | IGLV3-25 | down | 4.9 | 2.30E-05 | ---- |
| 224657_at | ERRFI1 | down | 3.8 | 1.02E-07 | ---- |
| 215379_x_at | IGLC1 | down | 3.7 | 1.03E-05 | ---- |
| 205239_at | AREG | down | 3.7 | 1.05E-03 | NM_001657 |
| 204533_at | CXCL10 | down | 3.7 | 1.98E-05 | NM_001565 |
| 214669_x_att | IGKC | down | 3.5 | 5.81 E-06 | ---- |
| 227372_s_at | t BAIAP2L1 | down | 3.5 | 3.58E-08 | AA496034 |
| 211430_s_at | t IGHG1 | down | 3.5 | 1.88E-06 | M87789 |
| 214836_x_at | IGKC, IGKV1-5 | down | 3.5 | 3.88E-05 | BG536224 |
| 208029 s at | LAPTM4B | down | 3.5 | 1.74E-05 | NM 018407 |
| 215223_s_at | SOD2 | down | 3.3 | 1.67E-06 | W46388 |
| 218280_x_at | HIST2H2AA | down | 3.3 | 3.53E-09 | ---- |
| 216834 at | RGS1 | down | 3.2 | 7.09E-04 | S59049 |
| 226452_at | PDK1 | down | 3.2 | 4.58E-10 | AU146532 |
| 216484_x_at | HDGF | down | 3.2 | 5.86E-10 | L24521 |
| 201596_x_at | KRT18 | down | 3.2 | 9.47E-11 | NM_000224 |
| 1554242_a_at | COCH | down | 3.2 | 6.10E-05 | --- |
| 208767_s_at | LAPTM4B | down | 3.1 | 1.22E-05 | AW149681 |
| 222449_at | TMEPAI | down | 3.0 | 3.23E-07 | ---- |
| 201037_at | PFKP | down | 3.0 | 5.78E-07 | NM 002627 |

| | | | | | |
|---|---|---|---|---|---|
| *Based on the probe set the gene can be identified in, for example, GeneAnnot (http://genecards.weizmann.ac.il/qeneannot/index.shtml. GeneAnnot provides a revised and improved annotation of Affymetrix probe-sets from HG-U95, HG-U133 and HG-U133 Plus2.0. Probe-sets are related to GeneCards genes, by direct sequence comparison of probes to GenBank, RefSeq and Ensembl mRNA sequences, while assigning sensitivity and specificity scores to each probe-set to gene match. (Ferrari F., Bortoluzzi S., Coppe A., Sirota A., Safran M., Shmoish M., Ferrari S., Lancet D., Danieli G.A., Bicciato S. Novel definition files for human GeneChips based on GeneAnnot. BMC Bioinformatics 8(1):446 (2007)). | | | | | |

## Claims

1. A method for diagnosing in an individual lung cancer or a predisposition for lung cancer, said method comprising determining in a sample taken from said individual differential expression of a panel of at least 2 and up to 50 different biomarkers selected from the biomarkers depicted in Table 1, Table 2, or both Table 1 and Table 2.

2. The method of claim 1, wherein differential expression is determined by way of comparison to a control sample.

3. The method of claims 1 or 2, comprising
i. determining in a biological sample of an individual suspected of suffering from lung cancer a target value for a panel of at least 2 and up to 50 different target biomarkers selected from the biomarkers depicted in Table 1, Table 2, or both Table 1 and Table 2;
ii. determining a reference value for said target biomarkers in a non-cancerous control sample;
iii. comparing the target value to the reference value; and
iv. predicting whether or not said individual has or is predisposed for lung cancer.

4. A method of distinguishing and classifying normal and tumor tissues comprising
i. determining in a tissue sample of an individual suspected of suffering from lung cancer a target value for the panel of target biomarkers selected from the biomarkers depicted in Table 1, Table 2, or both Table 1 and Table 2;
ii. determining a reference value for said target biomarkers in a non-cancerous control sample;
iii. comparing the target value to the reference value; and
iv. classifying the sample tissue as normal or tumor tissue.

5. The method according to any one of the preceding claims, wherein the sample is selected from blood, serum, plasma, sputum, saliva, tissue particularly lung tissue, obtained through biopsy, bronchia brushings, exhaled breath, or urine.

6. The method according to any one of the preceding claims, wherein determination of biomarker values is accomplished by performing an *in-vitro* assay, particularly an *in-vitro* assay selected from the group consisting of an antibody-based assay such as an immunoassay, a histological or cytological assay, an expression level assay such as an RNA expression level assay and an aptamer-based assay.

7. The method according to any one of the preceding claims, wherein the biomarker value is determined by performing
a. mass spectrometry;
b. an enzyme-linked immunosorbent assay;
c. a microarray-based immunohistochemical analysis; or
d. surface enhanced laser desorption/ionization.

8. The method according to any one of the preceding claims, wherein a polynucleotide or a variant thereof, which is complementary to a target gene as depicted in Table 1, Table 2, or both Table 1 and Table 2, is used as a molecular probe in a hybridization reaction or as a molecular primer in a nucleic acid extension reaction, for the determination of the target and reference value.

9. The method according to any one of the preceding claims, wherein one or more detectably labeled antibodies are used in the determination of the target and reference value, which antibodies are capable of identifying biomarker gene products encoded by one or more biomarker genes depicted in Table 1, Table 2, or both Table 1 and Table 2, or by conserved variants or peptide fragments thereof.

10. The method according to any one of the preceding claims, wherein a biomarker or a panel of biomarkers is used
(a) selected from the group of biomarkers depicted in Table 1 identified by GeneBank Accession number AI634580, NM_001611, NM_000669, D26054, AA551075, BG546884, NM_023504, AI382123, NM_002656, AW500340, AI694562, NM_000550. AA858297, BC001606, NM_001629, BE881590, AI680192, AW027333, and BF446673; or
(b) selected from the group of biomarkers depicted in Table 2 identified by GeneBank Accession number NM_001657, NM_001565, AA496034, M87789, BG536224, NM_018407, W46388, S59049, AU146532, L24521, NM_000224, AW149681, and NM_002627; or
(c) selected from the group of biomarkers depicted in Table 1 and Table 2 identified by GeneBank Accession number AI634580, NM_001611, NM_000669, D26054, AA551075, BG546884, NM_023504, AI382123, NM_002656, AW500340, AI694562, NM_000550. AA858297, BC001606, NM_001629, BE881590, AI680192, AW027333, BF446673, NM_001657, NM_001565, AA496034, M87789, BG536224, NM_018407, W46388, S59049, AU146532, L24521, NM_000224, AW149681, and NM_002627.

11. A panel of at least 2 biomarkers selected from the group of markers depicted in
Table 1, Table 2, or both Table 1 and Table 2 for use in a method according to any one of the preceding claims.

12. A panel of (a) at least 10 biomarkers selected from the group of markers depicted in Tables 1; or (b) at least 50 biomarkers selected from the group of markers depicted in Table 1, Table 2, or both Table 1 and Table 2 for use in a method according to any one of the preceding claims.

13. The panel of any one of claims 10 to 12, wherein said biomarker is a polynucleotide or a variant thereof, which is complementary to a target gene as depicted in Table 1, Table 2, or both Table 1 and Table 2 and can be used as a hybridization probe or a primer.

14. The panel of any one of claims 10 to 13, wherein said biomarkers are
(a) selected from the group of biomarkers depicted in Table 1 identified by GeneBank Accession number AI634580, NM_001611, NM_000669, D26054, AA551075, BG546884, NM_023504, AI382123, NM_002656, AW500340, AI694562, NM_000550. AA858297, BC001606, NM_001629, BE881590, AI680192, AW027333, and BF446673; or
(b) selected from the group of biomarkers depicted in Table 2 identified by GeneBank Accession number NM_001657, NM_001565, AA496034, M87789, BG536224, NM_018407, W46388, S59049, AU146532, L24521, NM_000224, AW149681, and NM_002627; or
(c) selected from the group of biomarkers depicted in Table 1 and Table 2 identified by GeneBank Accession number AI634580, NM_001611, NM_000669, D26054, AA551075, BG546884, NM_023504, AI382123, NM_002656, AW500340, AI694562, NM_000550. AA858297, BC001606, NM_001629, BE881590, AI680192, AW027333, BF446673, NM_001657, NM_001565, AA496034, M87789, BG536224, NM_018407, W46388, S59049, AU146532, L24521, NM_000224, AW149681, and NM_002627.

15. A kit for determining in an individual lung cancer or a predisposition for lung
cancer or for monitoring the progress of a lung cancer treatment in an individual, comprising reagent for detecting differential expression of a panel of at least 2 and up to 50 different biomarkers according to any one of claims 11 to 14.
